# EUROPÄISCHE PATENTSCHRIFT

(11) **EP 1 324 747 B1**
(45) Veröffentlichungstag und Bekanntmachung des Hinweises auf die Patenterteilung: **23.02.2005**
(21) Anmeldenummer: 01986598.9
(22) Anmeldetag: 05.10.2001
(51) Int. Cl.: A61K 7/48

(54) **KOSMETISCHE ZUBEREITUNG MIT PFLANZENEXTRAKTEN**
COSMETIC PREPARATION CONTAINING PLANT EXTRACTS
PREPARATION COSMETIQUE CONTENANT DES EXTRAITS DE PLANTES

(30) Priorität: 13.10.2000 DE 10053051
(43) Veröffentlichungstag der Anmeldung: 09.07.2003
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2001/003871
(87) Internationale Veröffentlichungsnummer: WO 2002/030384

(56) Entgegenhaltungen:
- EP-A- 0 279 392
- PATENT ABSTRACTS OF JAPAN vol. 18, no. 268 (C-1202), 23. Mai 1994 (1994-05-23) & JP 06 040933 A (NONOGAWA SHOJI KK), 15. Februar 1994 (1994-02-15)

## Beschreibung

Die Erfindung betrifft eine kosmetische Zubereitung mit einem Wirkkomplex aus Pflanzenextrakten.

Es sind bereits eine Vielzahl von kosmetischen Produkten beschrieben worden, die Pflanzen oder Pflanzenteile in direkter Verarbeitung oder als Extrakte enthalten. Zu diesen Pflanzen gehören beispielsweise Algen, verschiedenen als Heilpflanzen bekannte Kräuter, Getreidepflanzen, Tee-, Kaffee- und Früchte-Extrakte, Rindenextrakte sowie Hefe-Extrakte usw. Ziel des Einsatzes von Pflanzen ist die Nutzbarmachung der pflanzlichen Wirkstoffe für kosmetische Zwecke, insbesondere deren Wirkung in den oberen Hautschichten zu ermöglichen. Mit Ausnahme der Hefe-Extrakte sind bisher kaum Pilzextrakte für diesen Zweck eingesetzt worden, da auch kaum hautwirksame Stoffe aus diesem Bereich bekannt geworden sind.

Aus der EP 279392 ist der Einsatz von flüssigem Champagner mit einem Anteil von etwa 50-60 Gew-% an kosmetischen Präparaten bekannt, gegebenfalls zusammen mit einem tierischen Zellextrakt. In der JP 06-40933 A (Abstract) wird die Verwendung von Trüffelextrakten in Kosmetika beschrieben.

Der Erfindung liegt die Aufgabe zugrunde, weitere pflanzliche Wirkstoffe für die Kosmetik zu erschließen und insbesondere verschiedene Wirkstoffe unterschiedlicher pflanzlicher Herkunft zu neuen Wirkkomplexen zu vereinigen.

Erfindungsgemäß bereitgestellt wird eine kosmetische Zubereitung mit Pflanzenextrakten, die als Wirkkomplex wenigstens einen als wäßrigen Extrakt von echten Trüffeln (Tuberaceae) gewonnenen Ausgangsstoff enthält, der stabilisiert in einem kosmetisch annehmbaren Gel vorliegt.

Zu den echten Trüffeln gehören beispielsweise die Burgunder-Trüffel (Tuber uncinatum), Perigord-Trüffel (Tuber melanosporum Vitt.), Kalahari-Trüffel (Terfezia pfeilii Hennings), Piemont-Trüffel (Tuber magnatum Pico Vitt.), Löwen-Trüffel (Terfiza leonis Tul.), Sommer-Trüffel (Tuber aestivum), Winter-Trüffel (Tuber brumale Vitt.) und Weiße Trüffel (Choiromyces maeandriformis). Besonders bevorzugt sind Tuber uncinatum, Tuber melanosporum Vitt., Tuber magnatum Pico Vitt., Tuber aestivum, Choiromyces maeandriformis, Tuber brumale Vitt. und Gemische davon, insbesondere Tuber melanosporum Vitt.

Die echten Trüffel sind seit Jahrhunderten geschätzte Speisepilze aus der Klasse der Schlauchpilze (Ascomyten), Ordnung Tuberales, die wegen ihres besonderen Aromas eine hervorstechende Bedeutung erlangt haben. Diese Pilze wachsen unterirdisch in Symbiose mit Eichenwurzeln und bilden knollenförmige Fruchtkörper.

Bei der Trüffelextraktion werden verschiedene Wirkstoffe frei. Es wurde gefunden, daß durch Gehalte an essentiellen Aminosäuren, den Vitaminen B1, B2 und B3, Polysacchariden wie insbesondere Letinan und an Eritadenin in wäßrigem Trüffel-Extrakt und Stabilisierung dieser Wirkstoffe in einem Kosmetikum eine nicht-spezifische Stimulierung des Immunsystems und eine Verbesserung der antiviralen Abwehrkräfte des Organismus erreicht werden kann. Hautbezogen bedeutet dies eine ausgezeichnete Regenerativwirkung und darüber hinaus eine Wirkung gegen Haarausfall.

Ein besonderer Wirkkomplex entsteht durch Einbeziehung von Champagner-Produkten, wie z.B. einen in die feste Form, beispielsweise durch Sprühtrocknung, überführten Champagner. Auf Basis der speziellen Flaschengärung französischer Weine der Champagne wird ein Schaumwein erhalten, der hohe Anteile an Polysacchariden neben Flavonoiden, Pro-Cyanidolischen Oligomeren (P.C.O.) und Tanninen enthält. Die Einbeziehung in den Wirkkomplex mit Trüffel-Extrakt führt zu einer deutlich verbesserten Balance des Ökosystems der Haut, wodurch die natürliche Schutzbarriere der Haut verstärkt und die vorhandenen Mediatoren Leukotriene, Prostaglandine, Interleukine und andere Cytokinine verstärkt zur Anwendung gebracht werden, besonders bei Streßsituationen für die Haut.

Weiterhin zeigt der Wirkkomplex tonisierende und energetische Wirkungen und eine stabilisierende und erweichende Wirkung bei gleichzeitiger Anti-Irritationswirkung.

Der Wirkkomplex liegt stabilisiert in einem Gel vor. Die Stabilisierung kann z.B. durch ein Phospholipid erfolgen, in welchem die pulverförmigen Grundbestandteile des Wirkkomplexes Tuberaceae-Extrakt und gegebenenfalls Champagner-Produkt dispergiert werden, um dann anschließend in einem Gel homogen verteilt zu werden. Durch die homogene Verteilung in einem Gel erhält man eine Form des Wirkkoplexes, die einerseits eine stabile flüssige Form der Wirkstoffe darstellt und andererseits eine ausgezeichnete Basis für die Weiterverarbeitung zu verschiedenen kosmetischen Formen ist.

Als Phospholipide können z.B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phoslipon® oder NAT® .

Der Anteil des Wirkkomplexes in der kosmetischen Zubereitung kann im Bereich von 0,05 bis 25 Gew-% liegen, wobei die Anteile von Tuberaceae-Extrakt und gegebenenfalls Champagner-Produkt zusammen 1 bis 15 Gew-% betragen, bezogen auf den Wirkkomplex.

Der Tuberaceae-Extrakt und das Champagner-Produkt können in beliebigen verhältnissen in dem Wirkkomplex vorliegen. Bevorzugt ist das Verhältnis 25:75 bis 75:25, jeweils bezogen auf deren Trockenmasse.

Als Gele können solche Gelbildner eingesetzt werden, wie Carbomer, Xanthangummi, Carrageenan, Akaziengummi, Guargummi, Agar-Agar, Alginate und Tylosen, Carboxymethylcellulose, Hydroxyethylcellulose, bestimmte Polyacrylate, Polyvinylalkohol, Polyvinylpyrrolidon, Montmorillonit. Besonders bevorzugt sind Carbomer, Xanthangummi, Carrageenan, Akaziengummi, Guargummi, Agar-Agar, Alginate, Carboxymethylcellulose, Hydroxyethylcellulose und Gemische davon

Die erfindungsgemäße Zubereitung enthält weiterhin kosmetische Hilfs- und Trägerstoffe, wie sie üblicherweise in solchen Zubereitungen verwendet werden, z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Ester, Schellack, Elektrolyte, polare und unpolare öle, Polymere, Copolymere, Emulgatoren, Wachse, Stabilisatoren.

Weitere Zusatz- bzw. Wirkstoffe in den kosmetischen Zusammensetzungen können solche Vitamine sein, wie z.B. Vitamin A oder Vitamin A-Derivate, gefärbte Pflanzenextrakte, β-Caroten, organische wasserlösliche oder öllösliche Lichtschutzmittel wie z.B. Octylmethoxycinnamate; anorganische Lichtschutzfilter, wie TiO₂, ZnO, SiO₂.

Als weiteren Wirkstoff kann das Präparat vorteilhaft Kaolin gemäß WO96/17588 enthalten, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit. Der modifizierte Kaolin kann einen Anteil von 0,1 bis 6 Gew-% haben, bezogen auf die Gesamtmenge des Präparates.

Zu weiteren kosmetischen Wirkstoffen gehören z. B. Emulgatoren, Radikalfänger, Feuchthaltemittel, Enzyme, weitere pflanzliche Wirkstoffe, Polymere, Melanin, Antioxidationsmittel, entzündungswidrige natürliche Wirkstoffe, mit Sauerstoff beladene asymmetrische lamellare Aggregate gemäß WO 94/00109 oder Aufschlußprodukte von Hefen, Algen oder anderen pflanzlichen Stoffen, hergestellt durch ein schonendes Ultraschall-Aufschlußverfahren gemäß WO 94/13783.

Kosmetische Zubereitungen mit dem erfindungsgemäßen Wirkkomplex können als O/W- oder W/O-Emulsionen oder als Gel vorliegen.

Geeignete Emulgatoren für O/W-Emulsionen sind beispielsweise Anlagerungsprodukte von 2-30 Mol Ethylenoxid an lineare C₈-C₂₂-Fettalkohole, an C₁₂-C₂₂-Fettsäuren und an C₈-C₁₅-Alkylphenole; C₁₂-C₂₂-Fettsäuremono- und -diester von Anlagerungsprodukten von 1-30 Mol Ethylenoxid an Glycerin.

Geeignete Emulgatoren für W/O-Emulsionen sind beispielsweise Anlagerungsprodukte von 2-15 Mol Ethylenoxid an Ricinusöl; Ester von C₁₂-C₂₂-Fettsäuren und Glycerin, Polyglycerin, Pentaerythrit, Zuckeralkohole (z.B. Sorbit), Polyglucoside (z.B. Cellulose); Polyalkylenglycole; Wollwachsalkohole; Copolymere von Polysiloxan-Polyalkylpolyether.

Für die Erfindung geeignete öle und Fette für die Bildung der ölphase einer O/W- oder W/O-Emulsion sind beispielsweise Mineralöle, Fettsäuretriglyceride, Siliconöle sowie pflanzliche öle, wie Calendulaöl, Jojobaöl, Avocadoöl, Macadamianußöl, Rizinusöl, Weizenkeimöl, Babassuöl, Traubenkernöl, Kukuinußöl, Distelöl, Nachtkerzenöl, Safloröl oder ein Gemisch mehrerer davon.

Weiterhin können auch Ester oder Ether eingesetzt werden, wie zum Beispiel Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Butyl ether, Dicaprylyl ether, PPG1-PEG9 Lauroyl glycol ether, PPG15 Stearyl ether, PPG14 Butyl ether, Fomblin HC25.

Als weitere Erweichungsmittel können solche Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, mineralische öle, Butylmyristat, Palmitinsäure usw.

Die Verwendung des erfindungsgemäßen kosmetischen Präparates kann z.B. erfolgen in Sonnencremes, Sonnengelen, After-sun-Produkten, Tagescremes, Nachtcremes, Feuchtigkeitscremes, Masken, Körperlotionen, Reinigungsmilch, Make up's, Lippenstiften, Mascara, Pflegestiften, Körperpuder, Augenkosmetik, Haarmasken, Haarspülungen, Haarshampoos, Haarlotionen, Duschgelen, Seifen, Kompaktpudern. Die Herstellung derartiger Produkte erfolgt auf eine Weise, wie sie dem Fachmann auf diesem Gebiet bekannt ist.
Erfindungsgemäß erfolgt das Verfahren zur Herstellung einer kosmetischen Zubereitung mit Pflanzenextrakten dadurch, daß man a) Tuberaceae-Stücken mit Wasser über einen Zeitraum von 1,5 bis 12 Stunden vermischt,
b) das Gemisch für 0,5 bis 12 Stunden stehen läßt,
c) die abgetrennte wäßrige Phase in eine feste Form überführt und sterilisiert,
d) das sterilisierte Pulver in einem wäßrigen Phospholipid bei wenigstens 10000 bis etwa 15000 U/min dispergiert,
e) die Dispersion in einem kosmetisch annehmbaren Gel homogen verteilt und neutralisiert, und
f) das Gel mit weiteren kosmetischen Hilfsstoffen, Trägerstoffen oder Wirkstoffen oder Gemischen davon in Kontakt bringt und in eine kosmetische Anwendungsform formuliert.
Die Trennung des Extrakts kann durch Filtration erfolgen, gegebenenfalls durch gleichzeitige Entfärbung des Extraktes mittels z.B. Aktivkohle.

Die Erfindung soll nachstehend durch Beispiele näher erläutert werden. Alle Angaben erfolgen in Gewichtsprozent, sofern nichts anderes angegeben ist.

### Beispiel 1 bis 4

### Herstellung des Wirkkomplexes

a) 1 kg Perigord-Trüffel (Tuber melanosporum Vitt.) werden zerkleinert und mit 5 1 Wasser bei wenigstens 1000 U/min für 5 Stunden gerührt. Danach läßt man das Gemisch über Nacht stehen und filtriert anschließend ab. Das klare Filtrat wird auf einen Sprühtrockner gegeben, und das dabei erhaltene Pulver anschließend sterilisiert.
b) 10 Flaschen weißer Champagner werden sprühgetrocknet. Das dabei erhaltene weiße Pulver wird anschließend sterilisiert. Zur Herstellung des Wirkkomplexes werden der Trüffel-Extrakt gegebenenfalls zusammen mit einem gleichen Anteil des Champagner-Produktes in Wasser gegeben und zusammen mit einem wäßrigen Phospholipid (NAT80® ) unter Rühren mit wenigstens 10000 U/min und bei Raumtemperatur in eine Dispersion überführt. Anschließend wird die Dispersion mit dem wäßrigen Gel in Kontakt gebracht, gerührt und neutralisiert. Weiterhin können unter Rühren Glycerin und Ethanol zur besseren Verarbeitbarkeit zugegeben werden. Man erhält einen stabilisierten Wirkkomplex mit sehr guter Lagerfähigkeit.

| | Bsp.1 | Bsp.2 | Bsp.3 | Bsp.4 |
|---|---|---|---|---|
| **Bestandteile des Wirkkomplexes** | | | | |
| Wasser dest. | ad 100 | ad 100 | ad 100 | ad 100 |
| Carbomer | 0,5 | 0,5 | 0,5 | 0,2 |
| Glycerin | 6,0 | 6,0 | 6,0 | - |
| Konservierungsmittel | 0,9 | 0,9 | 0,9 | 1,0 |
| Triethanolamin | 0,5 | 0,5 | 0,5 | 0,5 |
| Ethanol | 6,0 | 3,0 | 1,0 | - |
| Tuber melanosporum Vitt. (Trockenextrakt) | 2,0 | 3,8 | 3,5 | - |
| Tuber aestivum (Tr.extrakt) | - | - | - | 4,0 |
| Champagner-Produkt (sprühgetrocknet) | 2,0 | 5,0 | 1,5 | - |
| Phospholipid | 8,0 | 8,0 | 8,0 | 5,0 |

### Beispiel 1a

Es wird die gleiche Zusammensetzung wie im Beispiel 1 verwendet, wobei der Trockenextrakt von Tuber melanosporum Vitt. 6 % beträgt und das getrocknete Champagner-Produkt 5 % beträgt.

### Beispiel 4a

Es wird die gleiche Zusammensetzung wie im Beispiel 4 verwendet, wobei der Trockenextrakt von Tuber aetivum 8 % beträgt und ein getrocknetes Champagner-Produkt mit 5 % eingesetzt wird.

### Beispiel 5 Creme I

| **Phase A** | |
|---|---|
| Wasser | ad 100 |
| Glycerin | 5,0 |
| Crosspolymere | 0,2 |

| **Phase B** | |
|---|---|
| Cetearyl Alcohol | 1,5 |
| Isohexadecane | 3,5 |
| Octyl Stearate | 3,0 |

| **Phase C** | |
|---|---|
| Triethanolamine (TEA) | 0,2 |

| **Phase D** | |
|---|---|
| Wirkkomplex gemäß Beispiel 1 | 2,0 |
| Konservierungsmittel | 0,5 |
| Parfümöl | 0,3 |

Phase A und Phase B werden separat unter Rühren auf 65 °C erwärmt und unter Rühren miteinander vereinigt. Die Temperatur wird auf etwa 50 °C verringert und die Phase C zugegeben. Bei etwa 35 °C wird unter Rühren die Phase D zugesetzt.

### Beispiel 6 Creme II

| **Phase A** | |
|---|---|
| Wasser | ad 100 |
| Polyacryamide | 1,5 |
| C13-14 Isoparaffin | 2,0 |
| Laureth 7 | 2,0 |

| **Phase B** | |
|---|---|
| Babassu Oil | 2,5 |
| Sesame Oil | 7,0 |
| Kaolin gemäß WO96/17588 Beisp. 1 | 5,0 |

| **Phase C** | |
|---|---|
| Wirkkomplex gemäß Beispiel 2 | 5,0 |
| Parfümöl | 0,5 |

Die getrennt hergestellten Phasen A und B wurden bei 40 ° unter Rühren miteinander vereinigt. Nach dem Abkühlen auf etwa 35 °C wurde die Phase C unter Rühren hinzugegeben.

### Beispiel 7 Sonnencreme

| **Phase A** | |
|---|---|
| Wasser | ad 100 |
| Propylen Glycol | 3,0 |
| PVM/MA Decadiene Crosspolymer | 0,5 |
| Benzophenone 3 | 2,5 |

| **Phase B** | |
|---|---|
| Octyl Stearate | 5,0 |
| Isohexadecane | 3,5 |
| Dimethicone | 1,0 |

| **Phase C** | |
|---|---|
| Pongamina extract (Pongamina pinnata) | 1,0 |
| Shellac | 1,0 |
| Octyl Methoxycinnamate | 7,5 |
| Butyl Methoxydibenzoylmethane | 2,0 |

| **Phase D** | |
|---|---|
| Wirkkomplex gemäß Beispiel 3 | 8,0 |
| Aloe vera | 0,5 |
| Konservierungsmittel | 0,5 |
| Perfümöl | 0,5 |

Phase A und Phase B werden separat unter Rühren auf 65 °C erwärmt und unter Rühren miteinander vereinigt. Die Temperatur wird auf etwa 60 °C verringert und die Phase C zugegeben. Bei etwa 35 °C wird unter Rühren die Phase D zugesetzt.

### Beispiel 8 Creme III

| **Phase A** | |
|---|---|
| Wasser | ad 100 |
| Glycerin | 5,5 |
| Crosspolymere | 0,2 |

| **Phase B** | |
|---|---|
| Cetearyl Alcohol | 1,8 |
| Isohexadecane | 3,2 |
| Octyl Stearate | 3,0 |

| **Phase C** | |
|---|---|
| TEA | 0,2 |

| **Phase D** | |
|---|---|
| Wirkkomplex gemäß Beispiel 4 | 10,0 |
| Konservierungsmittel | 0,5 |
| Parfümöl | 0,3 |

### Beispiel 9

Es wird eine Creme gemäß Beispiel 5 hergestellt mit einem Wirkkomplex gemäß Beispiel 1a, der einen Anteil von 10 % an der Gesamtzusammensetzung hat.

### Beispiel 10

Es wird eine Sonnencreme gemäß Beispiel 7 hergestellt mit einem Wirkkomplex gemäß Beispiel 4a, der einen Anteil von 12 % an der Gesamtzusammensetzung hat.

## Patentansprüche

1. Kosmetische Zubereitung mit Pflanzenextrakten, **dadurch gekennzeichnet, daß** sie als Wirkkomplex wenigstens einen als wäßrigen Extrakt von echten Trüffeln (Tuberaceae) gewonnenen Ausgangsstoff zusammen mit einem sprühgetrockneten Champagner enthält, wobei der Wirkkomplex stabilisiert in einem kosmetisch annehmbaren Gel vorliegt.

2. Zubereitung nach Anspruch 1, **dadurch gekennzeichnet, daß** die Tuberaceae ausgewählt sind unter Tuber uncinatum, Tuber melanosporum Vitt., Tuber magnatum Pico Vitt., Tuber aestivum, Choiromyces maeandriformis, Tuber brumale Vitt. und Gemischen davon.

3. Zubereitung nach Anspruch 1, **dadurch gekennzeichnet, daß** der Tuberaceae-Extrakt und das Champagner-Produkt im Verhältnis 25:75 bis 75:25 in dem Wirkkomplex vorliegen, jeweils bezogen auf deren Trockenmasse.

4. Zubereitung nach einem der Ansprüche 1 bis 3, **dadurch gekennzeichnet, daß** der in dem Gel stabilisierte wirkkomplex in einem Anteil von 0,05 bis 25 Gew-% vorliegt, bezogen auf die Gesamtzusammensetzung der Zubereitung, wobei die Anteile von Tuberaceae-Extrakt und Champagner in dem Wirkkomplex zusammen im Bereich von 1 bis 15 Gew-% liegen.

5. Zubereitung nach Anspruch 1, **dadurch gekennzeichnet, daß** das Stabilisierungsmittel ein Phospholipid umfaßt.

6. Zubereitung nach Anspruch 1, **dadurch gekennzeichnet, daß** das Gel ausgewählt ist unter Carbomer, Xanthangummi, Carrageenan, Akaziengummi, Guargummi, Agar-Agar, Alginaten, Carboxymethylcellulose, Hydroxyethylcellulose und Gemischen davon.

7. Zubereitung nach einem der Ansprüche 1 bis 5, **dadurch gekennzeichnet, daß** der Wirkkomplex zusätzlich wenigstens ein Schutzmittel gegen UV-Strahlung enthält.

8. Zubereitung nach Anspruch 7, **dadurch gekennzeichnet, daß** es als Creme, wie Tagescremes, Nachtcremes, Sonnencremes; Körperlotionen; Make up; Augenkosmetik, wie spezielle Augencreme, Lidschatten; Haarshampoos; Duschgel vorliegt.

9. Verfahren zur Herstellung.einer kosmetischen Zubereitung mit Pflanzenextrakten, **dadurch gekennzeichnet, daß** man
a) Tuberaceae-Stücken mit Wasser über einen Zeitraum von 1,5 bis 12 Stunden vermischt,
b) das Gemisch für 0,5 bis 12 Stunden stehen läßt,
c) die abgetrennte wäßrige Phase in eine feste Form überführt und sterilisiert,
d) das sterilisierte Pulver und einen sprühgetrockneter Champagner in einem wäßrigen Phospholipid bei wenigstens 10000 U/min dispergiert,
e) die Dispersion in einem kosmetisch annehmbaren Gel homogen verteilt und neutralisiert, und
f) das Gel mit weiteren kosmetischen Hilfsstoffen, Trägerstoffen oder Wirkstoffen oder Gemischen davon in Kontakt bringt und in eine kosmetische Anwendungsform formuliert.

## Claims

1. A cosmetic preparation containing plant extracts, which preparation comprises an active complex which is comprised of at least one basic material extracted as an aqueous extract from common truffles (Tuberaceae) together with a spray-dryed champagne, wherein the active complex is provided in a cosmetically acceptable gel while being stabilized.

2. A preparation according to Claim 1 wherein the Tuberaceae are selected from among Tuber uncinatum, Tuber melanosporum Vitt., Tuber magnatum Pico Vitt., Tuber aestivum, Choiromyces maeandriformis, Tuber brumale Vitt. and mixtures thereof.

3. A preparation according to Claim 1 wherein the Tuberaceae extract and the champagne product are contained in the active complex in a ratio ranging from 25:75 to 75:25 relative to the agents' dry weight.

4. A preparation according to any of Claims 1 through 3 wherein the active complex provided in the gel while being stabilized is contained in an amount ranging from 0.05 to 25% by weight relative to the overall composition of the preparation, the amounts of Tuberaceae extract and champagne jointly making up 1 to 15% by weight of the active complex.

5. A preparation according to Claim 1 wherein the stabilizer comprises a phospholipid.

6. A preparation according to Claim 1 wherein the gel is selected from among Carbomer, Xanthan Gum, Carrageenan, Acacia Gum, Guar Gum, Agar-Agar, alginates, carboxymethyl cellulose, hydroxyethyl cellulose and mixtures thereof.

7. A preparation according to any of Claims 1 through 6 wherein the active complex in addition contains at least one protective agent against UV radiation.

8. A preparation according to Claim 7 wherein the said preparation is provided in the form of a cream such as day creams, night creams, sun creams; body lotions; make-up; eye cosmetics such as special eye cream, eyeshadow; hair shampoos; shower gels.

9. Method for manufacturing a cosmetic preparation containing plant extracts, which method comprises the steps of
a) mixing pieces of Tuberaceae with water during a period of between 1.5 and 12 hours,
b) leaving the mixture to soak for 0.5 to 12 hours,
c) separating the aqueous phase, removing its liquid components and sterilizing it,
d) dispersing the sterilized powder and a spray-dried champagne in an aqueous phospholipid at at least 10,000 rpm,
e) distributing the dispersion in a cosmetically acceptable gel in a homogeneous manner and neutralizing it,
f) bringing the gel into contact with further cosmetic auxiliaries, carrier substances or active agents or mixtures thereof and formulating a cosmetic preparation.

## Revendications

1. Préparation cosmétique avec des extraits de plantes, **caractérisée en ce qu'**elle contient comme complexe actif, au moins une substance de départ obtenue sous la forme d'un extrait aqueux de vraies truffes (Tubéracées), avec un champagne séché par pulvérisation, où le complexe actif est présent sous forme stabilisée dans un gel cosmétiquement acceptable.

2. Préparation selon la revendication 1, **caractérisée en ce que** les Tubéracées sont choisies parmi *Tuber uncinatum, Tuber melanosporum Vitt., Tuber magnatum Pico Vitt., Tuber aestivum, Choiromyces maeandriformis, Tuber brumale Vitt.* et leurs mélanges.

3. Préparation selon la revendication 1, **caractérisée en ce que** l'extrait de Tubéracées et le produit du champagne sont présents en un rapport de 25:75 à 75:25 dans le complexe actif, chaque fois sur base de la masse sèche.

4. Préparation selon l'une quelconque des revendications 1 à 3, **caractérisée en ce que** le complexe actif stabilisé dans le gel est présent en une proportion de 0,05 à 25% en poids, sur base de la composition totale de la préparation, où la proportion d'extrait de Tubéracées et de champagne sont présents dans le complexe, ensemble, dans l'intervalle de 1 à 15% en poids.

5. Préparation selon la revendication 1, **caractérisée en ce que** l'agent de stabilisation comprend un phospholipide.

6. Préparation selon la revendication 1, **caractérisée en ce que** le gel est choisi parmi un carbomère, la gomme de xanthane, le carragheen, la gomme arabique, le gomme de guar, l'agarose, des alginates, la carboxyméthylcellulose, l'hydroxyéthyl-cellulose et leurs mélanges.

7. Préparation selon l'une quelconque des revendications 1 à 5, **caractérisée en ce que** le complexe actif contient en outre, au moins un agent protecteur contre les rayonnements U.V.

8. Préparation selon la revendication 7, **caractérisée en ce qu'**elle se présente sous la forme d'une crème, comme une crème de jour, une crème de nuit, une crème solaire ; une lotion pour le corps ; un maquillage ; un produit cosmétique pour les yeux, comme une crème spéciale pour les yeux, un fard à paupières ; un shampoing capillaire ; un gel douche.

9. Procédé de préparation d'une préparation cosmétique avec extraits de plantes, **caractérisé en ce que**
a) on mélange des morceaux de Tubéracées avec de l'eau pendant une période de 1,5 à 12 heures,
b) on laisse le mélange au repos pendant 0,5 à 12 heures,
c) la phase aqueuse séparée est convertie en une forme solide et stérilisée,
d) la poudre stérilisée et le champagne séché par pulvérisation sont dispersés dans un phospholipide aqueux à au moins 10 000 Tours/minute,
e) la dispersion est distribuée de manière homogène dans un gel cosmétiquement acceptable et neutralisée, et
f) le gel est mis en contact avec d'autres auxiliaires, matières support ou agents actifs cosmétiques, ou leurs mélanges, et formulé en une forme d'utilisation cosmétique.
